(19) Europäisches Patentamt
European Patent Office
Office européen des brevets

(11) **EP 3 257 933 A1**

(12) **EUROPEAN PATENT APPLICATION**
published in accordance with Art. 153(4) EPC

(43) Date of publication:
**20.12.2017 Bulletin 2017/51**

(21) Application number: **15882060.5**

(22) Date of filing: **24.12.2015**

(51) Int Cl.:
***C12M 1/00*** (2006.01)    ***C12N 15/09*** (2006.01)
***C12Q 1/68*** (2006.01)

(86) International application number:
**PCT/JP2015/085912**

(87) International publication number:
**WO 2016/129187 (18.08.2016 Gazette 2016/33)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **09.02.2015 JP 2015023478**

(71) Applicant: **Sony Corporation
Tokyo 108-0075 (JP)**

(72) Inventor: **MAEDA Tatsuo
Tokyo 108-0075 (JP)**

(74) Representative: **D Young & Co LLP
120 Holborn
London EC1N 2DY (GB)**

(54) **THERMAL CYCLER FOR REACTION OF BIOLOGICAL SUBSTANCE, BIOLOGICAL SUBSTANCE ANALYSIS DEVICE, SYSTEM FOR CONTROLLING TEMPERATURE CHANGE RATE IN REACTION OF BIOLOGICAL SUBSTANCE, METHOD FOR CONTROLLING TEMPERATURE CHANGE RATE IN REACTION OF BIOLOGICAL SUBSTANCE, BIOLOGICAL SUBSTANCE ANALYSIS METHOD, AND PROGRAM FOR CONTROLLING TEMPERATURE IN REACTION OF BIOLOGICAL SUBSTANCE**

(57)    A thermal cycler for analyzing a biological material is provided that provides excellent temperature control in biological material reaction cycles.

A thermal cycler for biological material is provided, including a heating/cooling unit capable of heating and/or cooling a biological material reaction region, and a control unit that controls an output power of the heating/cooling unit, in which the control unit controls the output power of the heating/cooling unit on the basis of a rate of temperature change of the biological material reaction region.

*FIG. 4*

**Description**

TECHNICAL FIELD

The present invention relates to a thermal cycler for biological material reaction, a biological material analysis apparatus, a system for controlling a rate of temperature change in a biological material reaction, a method for controlling a rate of temperature change in a biological material reaction, a biological material analysis method, and a temperature control program for biological material reaction.

BACKGROUND ART

In-vitro reaction or detection of a biological material in tissue or in a cell may sometimes require an operation of heating and cooling the subject biological material to specific temperatures.

An example of the case requiring such operation is nucleic acid amplification reaction. Examples of technology employing nucleic acid amplification reaction include a PCR method, reverse transcription polymerase chain reaction, a real-time PCR, DNA sequencing, a LAMP method, and the like.

For example, in a PCR method, a DNA polymerase and a primer are added to nucleic acid fragments to be amplified, and the mixture is heated to about 94°C to form a single strand (denaturation), and is then cooled to about 60°C. This causes a nucleic acid fragment and a primer complementary thereto to bind to each other to form a double strand (annealing). Then, the temperature is maintained between about 60°C and about 72°C, which causes a DNA complementary to the nucleic acid fragment to be synthesized starting at the point to which the primer binds, by an action of the DNA polymerase (elongation reaction). The elongation reaction is followed by repetition of a cycle in which the mixture is heated again for denaturation, is cooled for annealing, and is then maintained at an appropriate temperature for elongation reaction.

In recent years, temperature control in nucleic acid amplification reaction is provided primarily by using a Peltier element (e.g., Patent Documents 1 to 5).

CITATION LIST

PATENT DOCUMENT

Patent Document 1: Japanese Patent Application Laid-Open No. 2013-126421
Patent Document 2: Japanese Patent Application Laid-Open No. 2013-21988
Patent Document 3: Japanese Patent Application Laid-Open No. 2012-24042
Patent Document 4: Japanese Patent Application Laid-Open No. 2010-104385
Patent Document 5: Japanese Patent Application Laid-Open No. 2008-237207

SUMMARY OF THE INVENTION

PROBLEMS TO BE SOLVED BY THE INVENTION

However, temperature control using a Peltier element presents a problem in that the control varies from one Peltier element to another. This creates a need for adjustment of the drive voltage, and a countermeasure taken therefor, such as providing a power supply circuit dedicated for drive voltage adjustment, has increased the circuit size.

Moreover, another problem exists in that the degree of degradation of a Peltier element varies from one Peltier element to another. Thus, the control characteristic changes differently on an element-by-element basis. Moreover, the control characteristic continues to change until the degraded element is replaced.

It is a primary object of the present technology to provide a thermal cycler for biological material reaction that provides excellent temperature control in biological material reaction cycles.

SOLUTIONS TO PROBLEMS

To solve the above problem, the present technology provides a thermal cycler for biological material reaction, the thermal cycler including: a heating/cooling unit capable of heating and/or cooling a biological material reaction region; and a control unit configured to control an output power to the heating/cooling unit, in which the control unit controls the output power to the heating/cooling unit on the basis of a rate of temperature change of the biological material reaction region.

The control unit is capable, in a case where the heating/cooling unit degrades, and the rate of temperature change of the biological material reaction region is thus reduced, of increasing the output power of the heating/cooling unit to restore the rate of temperature change. In addition, in a case where the rate of temperature change of the biological material reaction region is high, the control unit is capable of reducing the output power of the heating/cooling unit to provide an appropriate rate of temperature change.

The rate of temperature change can be determined from a first temperature and a second temperature encountered during a transition of a temperature of the biological material reaction region from a first target temperature to a second target temperature.

In addition, the first temperature and the second temperature can be determined without using a predetermined initial and/or end period during the transition of the temperature of the biological material reaction region from the first target temperature to the second target temperature.

The present technology is also capable, in a case where the rate of temperature change becomes out of a predetermined criterion value range (falls below or exceeds a criterion value range), of controlling the output power of the heating/cooling unit so that the rate of temperature change falls within the predetermined criterion value range.

The present technology can further include: a temperature measurement unit configured to measure a temperature of the biological material reaction region. In addition, the present technology can include an alert unit that provides an alert in a case where the output power of the heating/cooling unit exceeds a predetermined output power.

In addition, the thermal cycler for biological material reaction of the present technology is usable for nucleic acid amplification reaction.

The present technology can also provide a nucleic acid analysis apparatus including a thermal cycler for biological material reaction including the heating/cooling unit and the control unit.

The present technology can also provide a control system for controlling a rate of temperature change in a biological material reaction including a heating/cooling apparatus and a control apparatus.

The control apparatus is capable of controlling an output power of the heating/cooling apparatus on the basis of a rate of temperature change of the biological material reaction region.

In the control system, at least a portion of apparatuses may be connected via a network.

In addition, the present technology can provide a method for controlling a rate of temperature change in a biological material reaction, including the steps of heating or cooling a biological material reaction region, and controlling an output power on the basis of a rate of temperature change of the biological material reaction region.

Further, the present technology can also provide a biological material analysis method including the steps of: heating or cooling a biological material reaction region; and controlling an output power on the basis of a rate of temperature change of the biological material reaction region.

The present technology can also provide a temperature control program used for controlling a rate of temperature change in a biological material reaction, the temperature control program for causing a computer to perform a control function that controls an output power of the heating/cooling unit on the basis of a rate of temperature change of a sample temperature in a biological material reaction region.

EFFECTS OF THE INVENTION

According to the present technology, a stable temperature change of a biological material reaction region is achievable in biological material reaction cycles.

Note that the effects described above are not limiting, and the effects may be any of the effects described in the present disclosure.

BRIEF DESCRIPTION OF DRAWINGS

Fig. 1 is a diagram illustrating an apparatus configuration of a thermal cycler for biological material reaction according to the present technology.
Fig. 2 is a diagram illustrating an example of a rate of temperature change of a biological material reaction region according to the present technology.
Fig. 3 is a diagram illustrating an example of a rate of temperature change of a biological material reaction region according to the present technology.
Fig. 4 is a diagram illustrating an example of a rate of temperature change of a biological material reaction region according to the present technology.
Fig. 5 is a flowchart of a temperature control program according to the present technology.

Fig. 6 is a flowchart of a temperature control program according to the present technology.
Fig. 7 is a flowchart of a temperature control program according to the present technology.
Fig. 8 is a flowchart of a temperature control program according to the present technology.

MODE FOR CARRYING OUT THE INVENTION

A preferred mode of practicing the present technology will be described below. It is to be understood that the embodiment described below is merely a representative embodiment of the present technology, and is not intended to restrict the scope of the present technology. The description is provided in the order set forth below:

1. Thermal cycler for biological material reaction

(1) Configuration of thermal cycler for biological material reaction

(a) Heating/cooling unit
(b) Control unit
(c) Other components

(c-1) Temperature measurement unit
(c-2) Alert unit

(2) Calculation of rate of temperature change of biological material reaction region

(a) Temperature change in biological material reaction region
(b) Temperature measurement point

(3) Correction of rate of temperature change of biological material reaction region

2. Biological material analysis apparatus including thermal cycler for biological material reaction
3. Control system for controlling rate of temperature change of biological material reaction region
4. Method for controlling rate of temperature change in biological material reaction
5. Biological material analysis method
6. Temperature control program for biological material reaction region

<1. Thermal cycler for biological material reaction>

(1) Configuration of thermal cycler for biological material reaction

The thermal cycler for biological material reaction (hereinafter also referred to simply as "thermal cycler") according to the present technology includes, at least, a heating/cooling unit capable of heating and/or cooling a biological material reaction region, and a control unit for an output power of the heating/cooling unit.

(a) Heating/cooling unit

The heating/cooling unit is a unit that heats and/or cools an area that serves as a reaction site of the biological material reaction. A reaction site is, for example, a site including a biological material, a reagent, a reactant, and the like, that exists in a reactor vessel, such as a tube, a microchip, etc. containing the biological material. The heating/cooling unit may heat and/or cool the reactor vessel itself to change the temperature of the reaction site via the reactor vessel.
Examples of specific device included in the heating/cooling unit include heating elements, such as a resistance heating element, a thermoelectric conversion element, and the like. Although there are no specific limitations in the present technology, a Peltier element is suitably used. This enables the biological material reaction region to be heated and/or cooled.

(b) Control unit

The control unit included in the present technology is a unit that controls an amount of current supplied to the heating/cooling unit.
A biological material reaction needs a suitable target heating temperature and a time required to reach that temperature, as well as a suitable target cooling temperature and a time required to reach that temperature.

In this regard, as described above, temperature control provided by a Peltier element, and/or the degree of degradation of a Peltier element, may vary from one Peltier element to another. Thus, the time required for the biological material reaction region to reach a target temperature may also vary. However, the time required to reach a target temperature is desired to be constant during a sequence of biological material reaction cycles.

In a case where the biological material reaction region is brought to a target temperature, the present technology can control the time required to reach that temperature by changing a maximum load current in the heating/cooling unit. In order that the maximum load current can be changed, the amount of current before degradation of the Peltier element is set to a slightly lower value to make an allowance in the initial magnitude of the current so that the current can be increased after degradation.

Note that, in the present technology, a difference between temperatures (temperature change) of a biological material reaction region in a unit time may be referred to as "rate of temperature change."

(c) Other components

Examples of other component included in the thermal cycler of the present technology include, for example, a temperature measurement unit and an alert unit, but are not limited thereto.

(c-1) Temperature measurement unit

Temperature measurement in the biological material reaction region of the thermal cycler may be performed by a known method, and for example, either a contact thermometer or a non-contact thermometer may be used. Examples thereof include use of a thermocouple thermometer, a laser for temperature measurement, and the like. Temperature measurement can be carried out using a combination of a heating element, a reactor vessel, a sample in the reactor vessel, and the like. Although no specific limitations exist on temperature measurement in the present technology, a suitable example is measuring a sample temperature.

(c-2) Alert unit

A suitable output power of the heating/cooling unit is a suitable maximum load current applicable to the heating/cooling unit. A suitable maximum load current may vary on the basis of a variation in the rate of temperature change. For example, a certain defect, such as degradation, occurring in a heating element included in the heating/cooling unit will cause an increase or decrease in the rate of temperature change, and hence an increase or decrease in the suitable maximum load current, accordingly. Then, in a case where the change in the maximum load current exceeds a specific value, it is preferable that, for example, the heating element be replaced. In such a situation, a suitable specific value or specific range of the maximum load current is previously determined, and in a case where a latest value of the maximum load current, which can be calculated in the method described below, exceeds the specific value or the specific range, an alert may be issued. If an alert unit is provided, the heating element can be replaced, or treated otherwise, in a timely manner when, for example, the heating element fails, or the heating element reaches an end of the service life because of the use of the thermal cycler, or for other reason.

An alert may be provided using a known method, examples of which include an alert using an indication on a display provided in or for the thermal cycler, an audible alert, and the like.

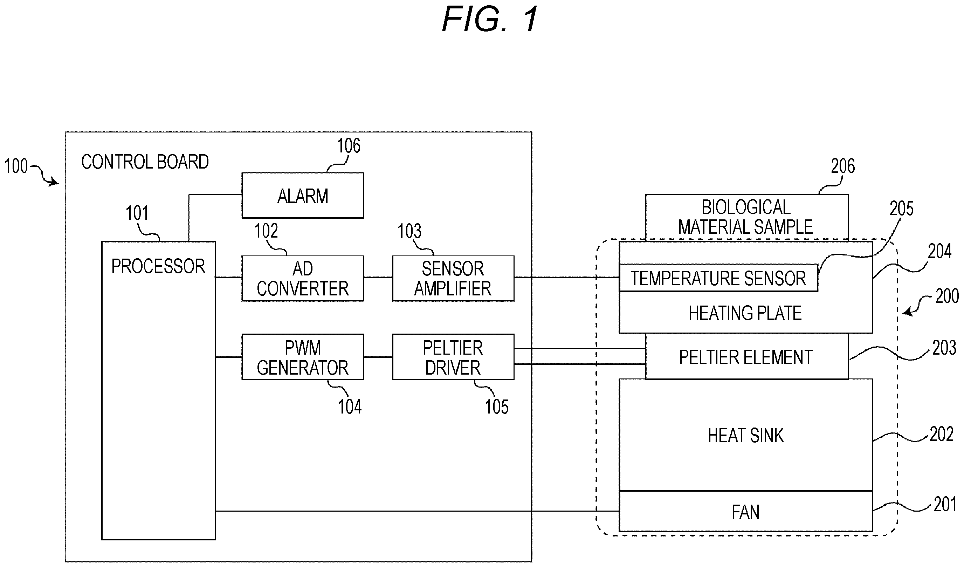

Fig. 1 illustrates one example of the configuration of the thermal cycler for biological material reaction according to the present technology.

A biological material sample 206 is temperature controlled by a Peltier element 203 via a heating plate 204. The Peltier element 203 is provided with a heat sink 202 and a fan 201.

In addition, a control board 100 includes a processor 101 mounted thereon. The processor 101 provides an instruction to heat or cool the biological material sample 206 by the Peltier element 203 via the heating plate 204. The processor 101 provides, at an appropriate time, an instruction to measure the temperature of the biological material sample 206, and a temperature sensor 205 measures the temperature of the biological material sample 206 via an AD converter 102 and a sensor amplifier 103.

In addition, the processor 101 adjusts a current using a PWM generator 104, and controls a current to the Peltier element 203 using a Peltier device driver 105. The current control is performed as follows: a rate of temperature change described below is calculated from a measurement result obtained in the temperature sensor 205; a suitable maximum load current to the Peltier element 203 is determined; and the processor 101 provides control so that the maximum load current will flow.

In Fig. 1, a dotted portion 200 including the Peltier element 203, the heating plate 204, etc., is a unit for heating or cooling the biological material sample 206. When the Peltier element etc. degrades, this unit 200 may be entirely replaced.

(2) Calculation of rate of temperature change of biological material reaction region

(a) Temperature change in biological material reaction region

A change in the temperature of the biological material reaction region (e.g., sample temperature) can be represented as the rate of temperature change.

The rate of temperature change is calculated, for example, by measuring the temperature of the biological material reaction region multiple times (e.g., twice). These temperature measurements are preferably performed during a temperature rise from an initial temperature (first target temperature) of the biological material reaction region to a second target temperature (e.g., about 94°C) for denaturation reaction in a biological material reaction cycle. In addition, temperature measurements may be performed during a temperature drop from a first target temperature (e.g., about 94°C) in denaturation reaction to a second target temperature (e.g., about 60°C) for annealing. Moreover, temperature measurements may be performed during a period from a first target temperature (e.g., about 60°C) for annealing to a second target temperature (e.g., about 65°C to about 72°C) for elongation reaction.

For example, in a case where the temperature is measured twice during a temperature rise for denaturation reaction of the biological material reaction region, the rate of temperature change can be calculated by dividing a difference between the second temperature (second measured temperature) and the first temperature (first measured temperature) by a time difference from the first measurement time to the second measurement time with respect to the beginning of the temperature rise as the starting point.

Note that the temperature measurements and/or the calculation of a rate of temperature change may be performed every biological material reaction cycle, every several cycles, or in a case where a PCR is performed once in 30 cycles of biological material reaction, only in the last cycle; and no specific limitations thus exist.

(b) Temperature measurement point

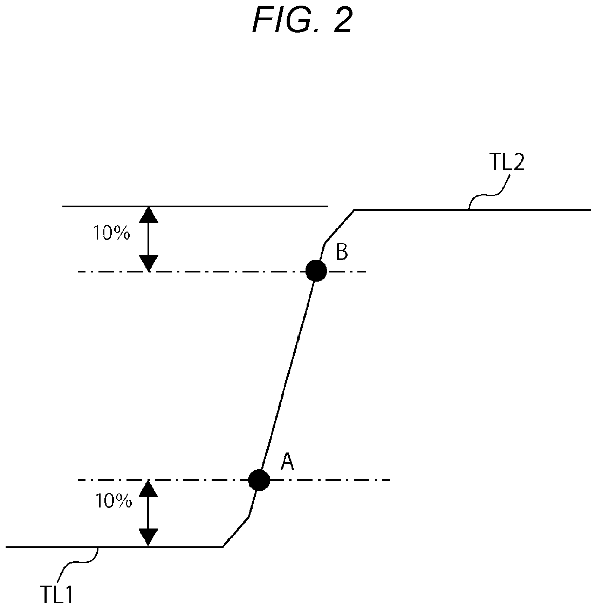

As shown in the graph of Fig. 2 for example, the temperature change of the biological material reaction region typically makes a curve, instead of increasing proportionally, in an initial period of a temperature rise and in an end period of the temperature rise immediately before reaching the target temperature.

Thus, in the present technology, the initial period and the end period of the temperature rise that may cause a curve may be excluded from the temperature measurements. That is, two or more temperature measurement points are set within a period of proportional temperature rise of the biological material reaction region. For example, a first and a second temperatures may be defined at the points indicated by circles (A, B) in Fig. 2 as the temperature measurement points.

Alternatively, the temperature measurement points may also be defined within a period of inversely proportional temperature drop of the biological material reaction region.

For example, the temperature measurement points may be defined as follows.

As shown in the graph of Fig. 2, defining the absolute value of the difference from a temperature before the temperature rise (first target temperature, TL1) to a temperature after the temperature rise (second target temperature, TL2) of the biological material reaction region, as 100%, a point that is 10% higher than the temperature at the beginning of the temperature rise is defined as a first temperature measurement point (A). Next, a point that is 10% lower than the temperature at the end of the temperature rise is defined as a second temperature measurement point (B).

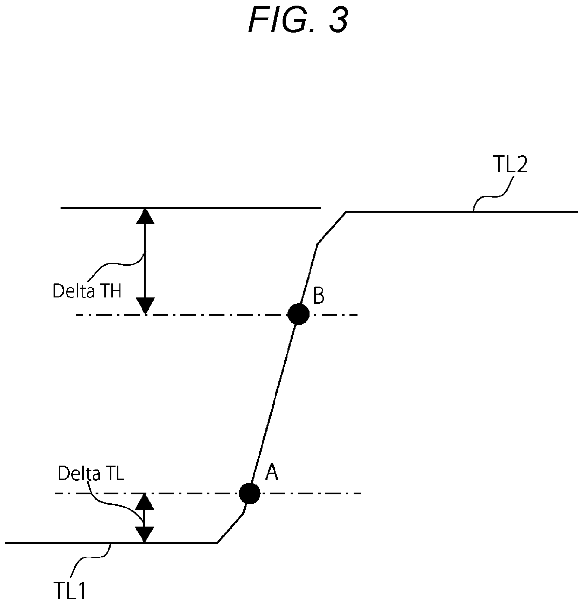

Alternatively, the temperature measurement points may be defined such that, for example, as shown in the graph of Fig. 3, a point higher by a predetermined difference (Delta TL) than the temperature at the beginning of the temperature rise of the biological material reaction region is a first temperature measurement point (A), while a point higher by a predetermined difference (Delta TH) than the temperature at the end of the temperature rise is a second temperature measurement point (B).

(3) Correction of rate of temperature change of biological material reaction region

The rate of temperature change of a biological material reaction region is preferably kept constant. However, for example, degradation of the heating element included in the heating/cooling unit may reduce the rate of temperature change in a biological material reaction region.

Assuming that the rate of temperature change of a biological material reaction region caused by a heating element before degradation (i.e., preferable target rate of temperature change) is 1, and that the rate of temperature change after degradation is 0.8, restoration of the rate of temperature change back to 1 will allow a sequence of temperature change cycles of the biological material reaction to proceed stably. Restoration of the rate of temperature change back to 1 can be provided by updating the load on the heating/cooling unit so that the output power of the heating/cooling unit (i.e., amount of current supplied to the heating/cooling unit) will be increased.

The amount of current to be supplied to the heating/cooling unit (updated maximum load current: Max Duty Update) can be calculated, for example, using a current maximum load current (Max Duty Current), a target rate of temperature change (Tilt Nominal), and a rate of temperature change (Tilt Measured) calculated after temperature measurement, using an equation shown below:

```
Equation 1

Max Duty Update = (Max Duty Current)*(Tilt

Nominal)/(Tilt Measured)
```

Applying to the heating/cooling unit the updated maximum load current calculated using the equation above enables, for example, the rate of temperature change to be adjusted from 0.8 to 1.

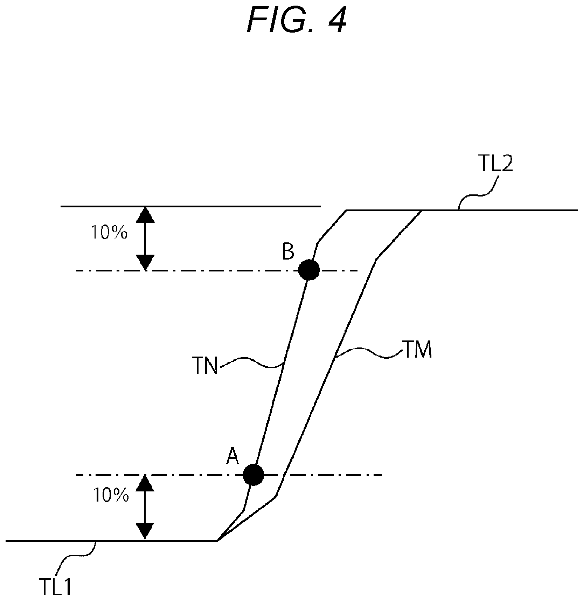

For example, in the graph of Fig. 4, the lower slope (TM: Tilt Measured) representing the calculated rate of temperature change of a degraded Peltier element can be changed to the higher slope (TN: Tilt Nominal) by increasing the maximum load current supplied to the heating/cooling unit.

In this regard, a criterion value may be predetermined (in this case, greater than or equal to 0.9 and less than or equal to 1, or with an allowance for the upper limit, greater than or equal to 0.9 and less than or equal to 1.1) so that, for example, in a case where the rate of temperature change falls below 0.9, the maximum load current obtained by the equation above is applied.

Note that even in a case where the rate of temperature change of the biological material reaction region exceeds a suitable target rate of temperature change, the rate of temperature change may also be restored by adjusting the maximum load current as described above.

Note that the technologies described in "(2) Calculation of rate of temperature change of biological material reaction region" provided above and in "(3) Correction of rate of temperature change of biological material reaction region" provided above are applicable to various methods that involve a reaction requiring a change in temperature, and may thus also be usable in nucleic acid amplification reaction cycles etc. used in a PCR method, a RT-PCR method, and the like.

<2. Biological material analysis apparatus including thermal cycler for biological material reaction>

Examples of the biological material analysis apparatus include nucleic acid analysis apparatuses, such as, for example, a microchip electrophoresis apparatus for DNA/RNA analysis, a gene detection apparatus, a genotyping apparatus, and a labeled nucleic acid quantification apparatus; an antigen-antibody reaction apparatus; a chromatography analysis apparatus; and the like. The thermal cycler utilizing the present technology may be provided upstream of these analysis apparatuses, and is not specifically limited.

Moreover, in addition to the thermal cycler and the biological material analysis apparatus, a device that serves as a biological material detection unit may further be included. The detection unit may include a detection device that utilizes a gene detection method using a conventional method, such as, for example, DNA sequencing, gel electrophoresis, hybridization using a DNA chip or bead, a real-time PCR method, or elongation reaction or hybridization using probe DNA; a detection method using a fluorescently labeled antibody; or the like.

<3. Control system for controlling rate of temperature change of biological material reaction region>

The control system for controlling a rate of temperature change of a biological material reaction region according to the present technology includes, at least, a heating/cooling apparatus capable of heating and/or cooling the biological material reaction region, and a control apparatus that controls an output power to the heating/cooling apparatus.

The heating/cooling apparatus is configured such that, for example, a biological material reactor vessel can be installed, and incorporates a heating element to heat and/or cool the vessel and/or a sample in the vessel. The heating element is preferably provided in the heating/cooling apparatus in an easily replaceable manner. As described earlier, a unit including components in addition to the heating element may be arranged so as to be entirely replaceable.

The control apparatus may include an apparatus, such as a computer, that executes a program for determining the maximum load current to be supplied to the heating/cooling apparatus, and thus provides temperature control. The details of the temperature control program will be described later herein.

The heating/cooling apparatus and the control apparatus may be connected to each other via a network. A sequence of cycles can be performed in which a suitable maximum load current, determined by the computer that may be included in the control apparatus, is supplied to the heating/cooling apparatus; the value of a latest suitable maximum load current for the heating/cooling apparatus is calculated anew by the computer that may be included in the control apparatus; and the latest suitable maximum load current is supplied to the heating/cooling apparatus.

The network may include a transmitter unit and a receiver unit for exchanging information between the heating/cooling apparatus and the control apparatus.

Moreover, in addition to the heating/cooling apparatus and the control apparatus, a biological material analysis apparatus, a display apparatus, an information processing apparatus, and/or the like, for example, may also be connected to one another via the network

<4. Method for controlling rate of temperature change in biological material reaction>

The method for controlling a rate of temperature change in a biological material reaction according to the present technology includes the steps of:

heating or cooling a biological material reaction region, and
controlling an output power to the heating/cooling unit on the basis of a rate of temperature change of the biological material reaction region.

The method for heating or cooling a biological material reaction region is not specifically limited. For example, when a Peltier element is used, supplying a direct current makes a temperature difference between both sides of the element, causing endothermic action in the lower temperature side, and exothermic action in the higher temperature side. Moreover, a reversal of the polarity of the current can change the heat transfer direction, and a change in the magnitude of the current can change the amount of heat. Thus, use of a Peltier element can provide both heating and cooling.

As described earlier, the method for measuring the temperature of a biological material reaction region is not specifically limited. In addition, the method for calculating the rate of temperature change is also as described above.

The method for controlling an output power of the heating/cooling unit may be performed by using a temperature regulator, or an operation device such as a power regulator. A temperature regulator receives a signal based on a rate of temperature change, and outputs an adjustment signal to the operation device. The operation device adjusts the current to be supplied to the heating/cooling unit.

<5. Biological material analysis method>

The biological material analysis method according to the present technology includes the steps of:

heating or cooling a biological material reaction region, and
controlling an output power to the heating/cooling unit on the basis of a rate of temperature change of the biological material reaction region.

Examples of a method for analyzing a biological material after performing these steps include, for example, sequencing of an amplification product using electrophoresis, a sequencer, or the like; microarray analysis; melting curve analysis performed in real-time PCR; and the like.

Moreover, in a case where the biological material is a DNA, and DNA profiling etc. is to be performed, the method may further include a step of analyzing a DNA profile.

Apparatuses incorporating software for performing nucleic acid analysis and/or DNA profiling are already commercially available, and any of such apparatuses may be combined with the thermal cycler of the present technology etc. to form a nucleic acid analysis apparatus as a whole.

<6. Temperature control program for biological material reaction region>

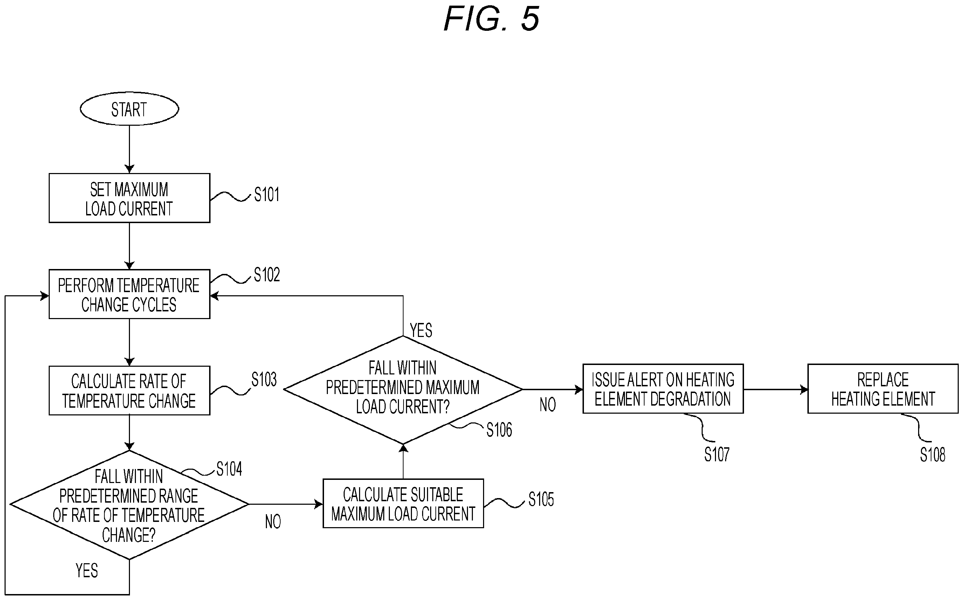

The temperature control program according to the present technology can be performed as shown in the flowchart shown in Fig. 5 as one example of the overall flow.

(i) First, a current maximum load current of the heating/cooling unit is set (S101). Here, a maximum load current indicative of a suitable rate of temperature change is set.
(ii) Next, in a biological material reaction region, a denaturation-annealing-elongation reaction cycle, that is, a cycle of temperature change involving, for example, a temperature of 94°C for denaturation, a temperature of 60°C for annealing, and a temperature of 70°C for elongation reaction, is repeated (S102).

(iii) Then, while the temperature is rising and/or falling toward a target temperature, measurements are made for the biological material reaction region multiple times as appropriate, and a rate of temperature change is calculated (S103).

(iv) Next, it is determined whether the calculated rate of temperature change is better than a predetermined value, or falls within a predetermined range, of the rate of temperature change (S104).

(v) In a case where the calculated rate of temperature change is better than the predetermined value, or falls within the predetermined range, of the rate of temperature change (YES), the cycle of temperature change is continuously repeated without change. In a case where the calculated rate of temperature change is worse than the predetermined value, or out of the predetermined range, of the rate of temperature change (NO), the maximum load current is calculated to restore the rate of temperature change to the initial suitable rate, and the maximum load current is change to the latest maximum load current (S105).

(vi) It is determined whether the latest maximum load current is better than a predetermined suitable value, or falls within a predetermined suitable range (S106).

(vii) In a case where the latest maximum load current is better than the predetermined suitable value, or falls within the predetermined suitable range, of the maximum load current (YES), the cycle of temperature change is continuously repeated without change. In a case where the latest maximum load current is worse than the predetermined suitable value, or out of the predetermined suitable range, of the maximum load current (NO), it is determined that the heating element is significantly degraded, and an alert is thus issued (S107).

(viii) When an alert is issued, the heating element is replaced (S108).

(ix) A maximum load current indicative of a suitable rate of temperature change for the new heating element is set (the process returns to step (i)), and then steps (ii) to (ix) are repeated.

The temperature control program of the present technology is recorded in an appropriate recording medium, and is executable by the computer, the heating/cooling unit, the control unit, and the like.

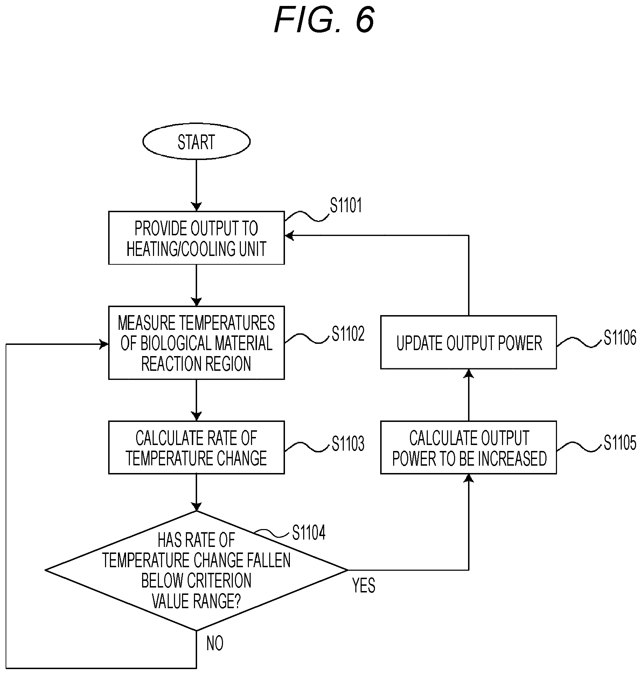

Fig. 6 illustrates a flowchart in a case where the Peltier element degrades.

(i) A maximum load current flows into the heating/cooling unit (S1101). The temperature of the heating/cooling unit rises, and the biological material reaction region is thus heated.

(ii) A first temperature and a second temperature of the biological material reaction region are measured (S1102).

(iii) A rate of temperature change is determined from the first temperature and the second temperature (S1103).

(iv) It is determined whether the rate of temperature change has fallen below a lower limit of a predetermined range (S1104). If the lower limit is not fallen below, the temperature of the biological material reaction region is measured every predetermined period of time (S1102), while if the lower limit is fallen below, calculation of an output power to be increased is made to cause the rate of temperature change to exceed the lower limit of the predetermined range (S1105).

(v) The output power is updated on the basis of the calculation result (S1106), and the output to the heating/cooling unit is increased. This can increase the output power, and hence the rate of temperature change, after degradation of the Peltier element. Therefore, even for repeated temperature change cycles, the temperature of the biological material reaction region can be stably changed.

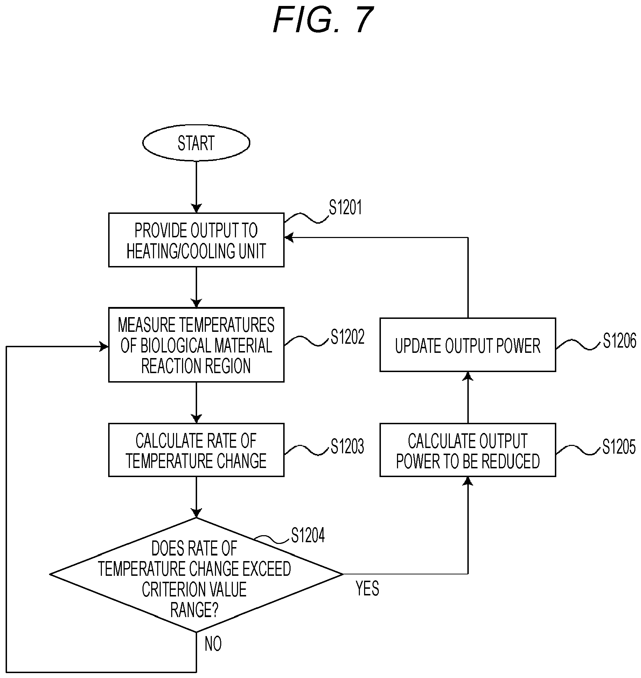

Fig. 7 illustrates a flowchart in a case where the temperature of the Peltier element rapidly rises.

(i) A maximum load current flows into the heating/cooling unit (S1201). The temperature of the heating/cooling unit rises, and the biological material reaction region is thus heated.

(ii) A first temperature and a second temperature of the biological material reaction region are measured (S1202).

(iii) A rate of temperature change is determined from the first temperature and the second temperature (S1203).

(iv) It is determined whether the rate of temperature change exceeds an upper limit of a predetermined range (S1204). If the upper limit is not exceeded, the temperature of the biological material reaction region is measured every predetermined period of time (S1202), while if the upper limit is exceeded, calculation of a output power to be reduced is made to cause the rate of temperature change to fall below the upper limit of the predetermined range (S1205).

(v) The output power is updated on the basis of the calculation result (S1206), and the output to the heating/cooling unit is reduced. This can reduce the output power, and hence the rate of temperature change, even when the Peltier element is overworking. Therefore, even for repeated temperature change cycles, the temperature of the biological material reaction region can be stably changed.

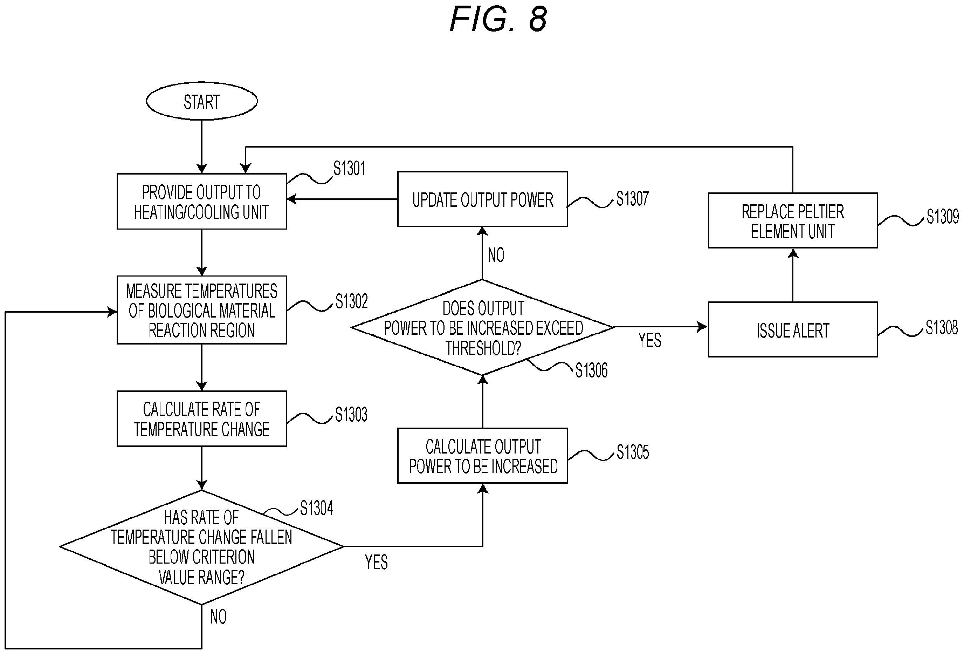

Fig. 8 illustrates a flowchart in a case where an alert procedure is included in the temperature control program.

(i) A maximum load current flows into the heating/cooling unit (S1301). The temperature of the heating/cooling unit increases, and the biological material reaction region is thus heated.

(ii) A first temperature and a second temperature of the biological material reaction region are measured (S1302).

(iii) A rate of temperature change is determined from the first temperature and the second temperature (S1303).

(iv) It is determined whether the rate of temperature change falls below a lower limit of a predetermined range (S1304). If the lower limit is not fallen below, the temperature of the biological material reaction region is measured every predetermined period of time (S1302), while if the lower limit is fallen below, calculation of an output power to be increased is made to cause the rate of temperature change to exceed the lower limit of the predetermined range (S1305).

(v) It is determined whether the output power to be increased exceeds a predetermined threshold (S1306). If the threshold is not exceeded (NO), the output power to the heating/cooling unit is updated to the calculated output power to be increased (S1307), while if the threshold is exceeded (YES), an alert unit issues an alert (S1308). The alert enables the apparatus user to know that the Peltier element is degraded, and that it is a time for replacement.

(vi) When an alert is issued, the Peltier element unit is replaced (S1309), and the flow is repeated from the step (i).

Note that the present technology can have the configurations described below.

[1] A thermal cycler for biological material reaction, the thermal cycler including:

a heating/cooling unit capable of heating and/or cooling a biological material reaction region; and
a control unit configured to control an output power to the heating/cooling unit,
in which the control unit controls the output power to the heating/cooling unit on the basis of a rate of temperature change of the biological material reaction region.

[2] The thermal cycler for biological material reaction according to [1], in which the rate of temperature change is determined from a first temperature and a second temperature encountered during a transition of a temperature of the biological material reaction region from a first target temperature to a second target temperature.

[3] The thermal cycler for biological material reaction according to [2], in which the first temperature and the second temperature are determined without using a predetermined initial and/or end period during the transition of the temperature of the biological material reaction region from the first target temperature to the second target temperature.

[4] The thermal cycler for biological material reaction according to any of [1] to [3], in which when the rate of temperature change of the biological material reaction region falls below a predetermined criterion value range, the heating/cooling unit has the output power increased so that the rate of temperature change falls within the predetermined criterion value range.

[5] The thermal cycler for biological material reaction according to any of [1] to [4], in which when the rate of temperature change of the biological material reaction region exceeds a predetermined criterion value range, the heating/cooling unit has the output power reduced so that the rate of temperature change falls within the predetermined criterion value range.

[6] The thermal cycler for biological material reaction according to any of [1] to [5], the thermal cycler further including:

a temperature measurement unit configured to measure a temperature of the biological material reaction region.

[7] The thermal cycler for biological material reaction according to any of [1] to [6], the thermal cycler further including:

an alert unit configured to provide an alert in a case where the output power to the heating/cooling unit exceeds a predetermined output power.

[8] The thermal cycler for biological material reaction according to any of [1] to [7], in which the biological material reaction is nucleic acid amplification reaction.

[9] A biological material analysis apparatus, including:

a thermal cycler for biological material reaction including

a heating/cooling unit capable of heating and/or cooling a biological material reaction region, and
a control unit configured to control an output power to the heating/cooling unit,
in which the control unit controls the output power to the heating/cooling unit on the basis of a rate of temperature change of the biological material reaction region.

[10] A control system for controlling a rate of temperature change in a biological material reaction, the system including:

a heating/cooling apparatus capable of heating and/or cooling a biological material reaction region, and
a control apparatus configured to control an output power to the heating/cooling apparatus,
in which the control apparatus controls the output power to the heating/cooling apparatus on the basis of a rate of temperature change of the biological material reaction region.

[11] The control system according to [10], in which at least a portion of apparatuses are connected via a network.
[12] A method for controlling a rate of temperature change in a biological material reaction, the method including the steps of:

heating or cooling a biological material reaction region; and
controlling an output power on the basis of a rate of temperature change of the biological material reaction region.

[13] A biological material analysis method including the steps of:

heating or cooling a biological material reaction region; and
controlling an output power on the basis of a rate of temperature change of the biological material reaction region.

[14] A temperature control program used for controlling a rate of temperature change in a biological material reaction, the program for causing a computer to perform control function that controls an output power on the basis of a rate of temperature change of a biological material reaction region.

**Claims**

1.  A thermal cycler for biological material reaction, the thermal cycler comprising:

    a heating/cooling unit capable of heating and/or cooling a biological material reaction region; and
    a control unit configured to control an output power to the heating/cooling unit,
    wherein the control unit controls the output power to the heating/cooling unit on the basis of a rate of temperature change of the biological material reaction region.

2.  The thermal cycler for biological material reaction according to claim 1, wherein the rate of temperature change is determined from a first temperature and a second temperature encountered during a transition of a temperature of the biological material reaction region from a first target temperature to a second target temperature.

3.  The thermal cycler for biological material reaction according to claim 2, wherein the first temperature and the second temperature are determined without using a predetermined initial and/or end period during the transition of the temperature of the biological material reaction region from the first target temperature to the second target temperature.

4.  The thermal cycler for biological material reaction according to claim 1, wherein when the rate of temperature change of the biological material reaction region falls below a predetermined criterion value range, the heating/cooling unit has the output power increased so that the rate of temperature change falls within the predetermined criterion value range.

5.  The thermal cycler for biological material reaction according to claim 1, wherein when the rate of temperature change of the biological material reaction region exceeds a predetermined criterion value range, the heating/cooling unit has the output power reduced so that the rate of temperature change falls within the predetermined criterion value range.

6.  The thermal cycler for biological material reaction according to claim 1, the thermal cycler further comprising:

    a temperature measurement unit configured to measure a temperature of the biological material reaction region.

7.  The thermal cycler for biological material reaction according to claim 1, the thermal cycler further comprising:

an alert unit configured to provide an alert in a case where the output power to the heating/cooling unit exceeds a predetermined output power.

8. The thermal cycler for biological material reaction according to claim 1, wherein the biological material reaction is nucleic acid amplification reaction.

9. A biological material analysis apparatus, comprising:

a thermal cycler for biological material reaction including

a heating/cooling unit capable of heating and/or cooling a biological material reaction region, and
a control unit configured to control an output power to the heating/cooling unit,
wherein the control unit controls the output power to the heating/cooling unit on the basis of a rate of temperature change of the biological material reaction region.

10. A control system for controlling a rate of temperature change in a biological material reaction, the system comprising:

a heating/cooling apparatus capable of heating and/or cooling a biological material reaction region, and
a control apparatus configured to control an output power to the heating/cooling apparatus,
wherein the control apparatus controls the output power to the heating/cooling apparatus on the basis of a rate of temperature change of the biological material reaction region.

11. The control system according to claim 10, wherein at least a portion of apparatuses are connected via a network.

12. A method for controlling a rate of temperature change in a biological material reaction, the method comprising the steps of:

heating or cooling a biological material reaction region; and
controlling an output power on the basis of a rate of temperature change of the biological material reaction region.

13. A biological material analysis method comprising the steps of:

heating or cooling a biological material reaction region; and
controlling an output power on the basis of a rate of temperature change of the biological material reaction region.

14. A temperature control program used for controlling a rate of temperature change in a biological material reaction, the program for causing a computer to perform control function that controls an output power on the basis of a rate of temperature change of a biological material reaction region.

EP 3 257 933 A1

*FIG. 1*

CONTROL BOARD

100

ALARM — 106

PROCESSOR — 101

AD CONVERTER — 102

SENSOR AMPLIFIER — 103

PWM GENERATOR — 104

PELTIER DRIVER — 105

BIOLOGICAL MATERIAL SAMPLE — 206

TEMPERATURE SENSOR — 205

HEATING PLATE — 204

200

PELTIER ELEMENT — 203

HEAT SINK — 202

FAN — 201

# FIG. 2

# FIG. 3

TL2

Delta TH

B

Delta TL

A

TL1

## FIG. 4

*FIG. 5*

# FIG. 6

# FIG. 7

```
                    ( START )
                        |
                        v
        +----------------------------+        S1201
        |   PROVIDE OUTPUT TO        |<-------+
        |   HEATING/COOLING UNIT     |        |
        +----------------------------+        |
                        |                     |
                        v                     |
        +----------------------------+   S1202 |  +----------------------+  S1206
        |   MEASURE TEMPERATURES     |         |  |  UPDATE OUTPUT POWER  |
    +-->|   OF BIOLOGICAL MATERIAL   |         |  +----------------------+
    |   |   REACTION REGION          |         |            ^
    |   +----------------------------+         |            |
    |                   |                      |            |
    |                   v                      |            |
    |   +----------------------------+  S1203   |  +----------------------+ S1205
    |   |   CALCULATE RATE OF        |          |  | CALCULATE OUTPUT     |
    |   |   TEMPERATURE CHANGE       |          |  | POWER TO BE REDUCED  |
    |   +----------------------------+          |  +----------------------+
    |                   |                       |            ^
    |                   v           S1204        |            |
    |            /\                              |            |
    |          /    \                           |            |
    |    DOES RATE OF                            |            |
    |  TEMPERATURE CHANGE EXCEED  \  ---YES-----+            |
    |   \  CRITERION VALUE      /                            |
    |     \     RANGE?        /                              |
    |       \              /                                 |
    |         \  NO  /                                       |
    |            |                                           |
    +------------+
```

## FIG. 8

START

PROVIDE OUTPUT TO HEATING/COOLING UNIT — S1301

MEASURE TEMPERATURES OF BIOLOGICAL MATERIAL REACTION REGION — S1302

CALCULATE RATE OF TEMPERATURE CHANGE — S1303

HAS RATE OF TEMPERATURE CHANGE FALLEN BELOW CRITERION VALUE RANGE? — S1304
NO
YES

CALCULATE OUTPUT POWER TO BE INCREASED — S1305

DOES OUTPUT POWER TO BE INCREASED EXCEED THRESHOLD? — S1306
NO
YES

UPDATE OUTPUT POWER — S1307

ISSUE ALERT — S1308

REPLACE PELTIER ELEMENT UNIT — S1309

EP 3 257 933 A1

## INTERNATIONAL SEARCH REPORT

| International application No. |
|---|
| PCT/JP2015/085912 |

**A. CLASSIFICATION OF SUBJECT MATTER**
*C12M1/00*(2006.01)i, *C12N15/09*(2006.01)i, *C12Q1/68*(2006.01)i

According to International Patent Classification (IPC) or to both national classification and IPC

**B. FIELDS SEARCHED**

Minimum documentation searched (classification system followed by classification symbols)
C12M1/00, C12N15/09, C12Q1/68

Documentation searched other than minimum documentation to the extent that such documents are included in the fields searched
Jitsuyo Shinan Koho      1922-1996   Jitsuyo Shinan Toroku Koho   1996-2016
Kokai Jitsuyo Shinan Koho   1971-2016   Toroku Jitsuyo Shinan Koho   1994-2016

Electronic data base consulted during the international search (name of data base and, where practicable, search terms used)
JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
|---|---|---|
| X  Y | Kazuhiko AOYANAGI, PCR no Sochi, Experimental Medicine separate volume Mokutekibetsu de Eraberu PCR Jikken Protocol, 1st print, Yodosha Co., Ltd., 01 November 2011 (01.11.2011), pages 19 to 25, page 19, line 1 to page 20, line 5, table 1, fig. 1 to 3 | 1,2,8-10, 12-14  1-14 |
| X  Y | JP 2013-523165 A (Life Technologies Corp.), 17 June 2013 (17.06.2013), particularly, claims; paragraphs [0002], [0030] to [0041]; drawings & WO 2011/127386 A2 particularly, claims; paragraphs [0002], [0057] to [0068]; drawings & US 2011/0275055 A1    & EP 2556173 A & SG 184539 A           & CN 103003448 A | 1,2,8-10, 12-14  1-14 |

☒  Further documents are listed in the continuation of Box C.    ☐  See patent family annex.

| | |
|---|---|
| *      Special categories of cited documents:<br>"A"   document defining the general state of the art which is not considered    to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| 08 March 2016 (08.03.16) | 22 March 2016 (22.03.16) |

| Name and mailing address of the ISA/<br>Japan Patent Office<br>3-4-3,Kasumigaseki,Chiyoda-ku,<br>Tokyo 100-8915,Japan | Authorized officer<br><br><br>Telephone No. |
|---|---|

Form PCT/ISA/210 (second sheet) (January 2015)

Form PCT/ISA/210 (continuation of second sheet) (January 2015)

**INTERNATIONAL SEARCH REPORT**

| International application No. |
|---|
| PCT/JP2015/085912 |

C (Continuation).  DOCUMENTS CONSIDERED TO BE RELEVANT

| Category* | Citation of document, with indication, where appropriate, of the relevant passages | Relevant to claim No. |
|---|---|---|
| X | JP 2008-237207 A  (Sony Corp.),<br>09 October 2008 (09.10.2008), | 1,2,8-10,<br>12-14 |
| Y | particularly, claims; paragraphs [0037], [0038],<br>[0057]; drawings<br>& US 2008/0220509 A1<br>paragraphs [0043], [0044], [0063]; drawings<br>& JP 2010-104385 A       & EP 1964610 A2<br>& CN 101255396 A | 1-14 |
| Y | WO 2006/067838 A1  (Advantest Corp.),<br>29 June 2006 (29.06.2006),<br>particularly, claims; paragraphs [0007] to<br>[0011], [0030] to [0039], [0046] to [0047];<br>drawings<br>& US 2008/0106293 A1<br>particularly, claims; paragraphs [0007] to<br>[0011], [0032] to [0041], [0048] to [0049];<br>drawings | 1-14 |
| Y | JP 5-241668 A  (Oki Electric Industry Co.,<br>Ltd.),<br>21 September 1993 (21.09.1993),<br>particularly, claims; paragraphs [0020] to<br>[0036]; drawings<br>(Family: none) | 1-14 |
| Y | JP 2001-15819 A  (Sunx Ltd.),<br>19 January 2001 (19.01.2001),<br>particularly, claims; paragraphs [0005] to<br>[0006], [0011]; drawings<br>(Family: none) | 1-14 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013126421 A
- JP 2013021988 A
- JP 2012024042 A
- JP 2010104385 A
- JP 2008237207 A